(19) Europäisches Patentamt / European Patent Office / Office européen des brevets

(11) **EP 2 994 043 B1**

(12) **FASCICULE DE BREVET EUROPEEN**

(45) Date de publication et mention de la délivrance du brevet:
**31.07.2019 Bulletin 2019/31**

(51) Int Cl.:
*A61B 5/00* *(2006.01)*   *A61B 5/021* *(2006.01)*
*A61B 5/02* *(2006.01)*

(21) Numéro de dépôt: **14729947.3**

(22) Date de dépôt: **29.04.2014**

(86) Numéro de dépôt international:
**PCT/FR2014/051026**

(87) Numéro de publication internationale:
**WO 2014/181056 (13.11.2014 Gazette 2014/46)**

(54) **APPAREIL ET PROCEDE DE DETERMINATION DE LA VITESSE DE PROPAGATION D'UNE ONDE DE POULS**

VORRICHTUNG UND VERFAHREN ZUR BESTIMMUNG DER AUSBREITUNGSGESCHWINDIGKEIT EINER PULSWELLE

APPARATUS AND METHOD FOR DETERMINING THE PROPAGATION SPEED OF A PULSE WAVE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.05.2013 FR 1354131**

(43) Date de publication de la demande:
**16.03.2016 Bulletin 2016/11**

(73) Titulaire: **Hallab, Magid**
**44460 Saint Nicolas de Redon (FR)**

(72) Inventeur: **Hallab, Magid**
**44460 Saint Nicolas de Redon (FR)**

(74) Mandataire: **Ermeneux, Bertrand**
**AVOXA**
**5 allée Ermengarde d'Anjou**
**ZAC Atalante Champeaux**
**CS 40824**
**35108 Rennes (FR)**

(56) Documents cités:
**FR-A1- 2 947 167      US-A1- 2008 027 330**

- **M. HALLAB ET AL: "Un nouvel index pour évaluer le vieillissement artériel indépendamment de la pression artérielle : pOpscore", ANNALES DE CARDIOLOGIE ET D'ANGÉIOLOGIE, vol. 61, no. 3, 8 mai 2012 (2012-05-08), pages 184-187, XP055099934, ISSN: 0003-3928, DOI: 10.1016/j.ancard.2012.04.014**
- **JUAN M PADILLA ET AL: "Pulse Wave Velocity and Digital Volume Pulse as Indirect Estimators of Blood Pressure: Pilot Study on Healthy Volunteers", CARDIOVASCULAR ENGINEERING: AN INTERNATIONAL JOURNAL, KLUWER ACADEMIC PUBLISHERS-PLENUM PUBLISHERS, NE, vol. 9, no. 3, 6 août 2009 (2009-08-06), pages 104-112, XP019728147, ISSN: 1573-6806, DOI: 10.1007/S10558-009-9080-5**
- **NITZAN M ET AL: "The difference in pulse transit time to the toe and finger measured by photoplethysmography; Difference in pulse transit time to the toe and finger", PHYSIOLOGICAL MEASUREMENT, INSTITUTE OF PHYSICS PUBLISHING, BRISTOL, GB, vol. 23, no. 1, 19 décembre 2001 (2001-12-19), pages 85-93, XP020073583, ISSN: 0967-3334, DOI: 10.1088/0967-3334/23/1/308**
- **MAGID HALLAB ET AL: "Relationship between the aortic valves and an anatomical landmark using chest CT scan", ARTERY RESEARCH, vol. 6, no. 1, 1 mars 2012 (2012-03-01), pages 55-57, XP055141870, ISSN: 1872-9312, DOI: 10.1016/j.artres.2011.09.001**

EP 2 994 043 B1

# Description

La présente invention concerne de manière générale la détermination de la vitesse de propagation d'une onde de pression sanguine, dite onde de pouls, d'un sujet.

La vitesse de propagation de l'onde de pouls est un paramètre qui permet d'apprécier l'état physique des artères à travers lesquelles se propage ladite onde, en vue d'estimer le risque cardio-vasculaire chez un sujet. Le risque d'accident cardio-vasculaire peut se traduire notamment par les risques suivants pour les organes du sujet :

- le coeur : angor, infarctus ;
- le cerveau : hémorragie cérébrale ou ischémie ;
- le rein : insuffisance rénale ;
- l'aorte : anévrisme, dissection.

On connaît de l'état de la technique des procédés de détermination de la vitesse de propagation de l'onde de pouls utilisés pour déterminer un indice d'onde de pouls fonction de la vitesse d'onde de pouls mesurée entre le coeur et un doigt, et de la vitesse d'onde de pouls mesurée entre le coeur et un orteil. Un tel procédé est décrit dans la demande de brevet publiée sous le numéro FR2947167 et permet de déterminer un éventuel risque cardio-vasculaire.

On connait également du document US-A1-2008/027330 une méthode de détermination de la vitesse de propagation de l'onde de pouls entre le coude et le poignet d'un sujet et de l'article Padilla et al. « Pulse Wave Velocity and Digital Volume Pulse as indirect estimators of blood pressure: pilot study on healthly volunteers", cardiovascular engineering, Kluwer academic publishers- Plenum publishers, NE, vol. 9, n°3, 2009, une méthode de détermination de la vitesse de propagation de l'onde de pouls entre le bras et la cheville d'un sujet.

On connait par ailleurs de l'article Hallab et al. « un nouvel index pour évaluer le vieillissement artériel indépendamment de la pression artérielle : pOpscore » , Annales de cardiologie et d'angéiologie, vol. 61, n°3, 2012, un procédé de détermination de la vitesse de propagation de l'onde de pouls dans l'aorte mesurée entre un doigt et un orteil d'un sujet, prenant en compte la taille du sujet pour calculer la longueur aortique.

Une étude de l'évolution du temps de transit d'une onde de pouls entre un doigt et un orteil d'un sujet en fonction de l'âge du sujet est en outre connue de l'article Nitzan et al. « The difference in pulse transit time to the toe and finger measured by photoplethysmography: difference in pulse transit time to the toe and finger ", Physiological measurement, institute of physics publishing, vol. 23, n°1, 2001.

Pour améliorer encore la détermination de risques liés à la rigidité artérielle, il est souhaitable de pouvoir utiliser un autre procédé et un autre appareil correspondant, c'est-à-dire un procédé et un appareil alternatifs à la solution connue de l'état de la technique, tout en conservant une fiabilité et une répétabilité des mesures.

Le but de la présente invention est ainsi de proposer un appareil et un procédé, alternatifs à la solution connue de l'état de la technique, qui permettent de déterminer de manière fiable et répétable un paramètre physique d'un sujet dont la valeur varie de manière fiable avec la rigide artérielle dudit sujet.

Un autre but de l'invention est de proposer un appareil et un procédé de détermination de la vitesse de propagation d'une onde de pouls, aisés et rapides à utiliser ou à mettre en oeuvre, même pour une personne ne possédant pas d'expertise médicale.

A cet effet, l'invention a pour objet un appareil de détermination de la vitesse de propagation d'une onde de pression sanguine d'un sujet, dite onde de pouls, ledit appareil comprenant, soit un capteur d'onde de pouls, soit un premier et un deuxième capteur d'onde de pouls, ledit ou chaque capteur étant positionnable à un doigt ou à un orteil du sujet, ledit appareil comprenant aussi une unité de traitement et de calcul configurée pour :

- déterminer l'instant d'arrivée d'une onde de pouls à un doigt du sujet, lorsque ledit ou l'un desdits capteur(s) est positionné audit doigt ;
- déterminer l'instant d'arrivée d'une onde de pouls à un orteil du sujet, lorsque ledit ou l'un desdits capteur(s) est positionné audit orteil ;
- calculer une valeur de temps, appelée durée de transit aortique, correspondant à la différence entre lesdits instants d'arrivée ;
- calculer ladite vitesse de propagation d'onde de pouls, appelée vitesse d'onde de pouls aortique, en fonction de ladite durée de transit aortique, ledit calcul comprenant la division de la longueur aortique (Laort), calculée par ladite unité de traitement et de calcul à partir de la taille (H) et de l'âge du sujet, par ladite durée de transit aortique (Top_aort),

ledit appareil comprenant en outre lorsqu'il comprend un seul capteur d'onde de pouls, des moyens de mesure de l'activité cardiaque (2) du sujet destinés à permettre une détection de l'instant de départ (TA) de l'onde de pouls.

Autrement dit, la vitesse d'onde de pouls aortique est obtenue par un calcul prenant en compte la différence de temps d'arrivée de l'onde de pouls entre un orteil et un doigt et une estimation de la longueur aortique dépendant de la taille et de l'âge du sujet.

Le demandeur a en effet pu constater à travers des essais que la longueur aortique est sensiblement proportionnelle à la taille du sujet et varie en fonction de l'âge du sujet.

Comme détaillé ci-après, le fait de calculer une valeur de temps fonction de la différence entre l'instant d'arrivée d'une onde de pouls à l'orteil et l'instant d'arrivée d'une onde de pouls au doigt, permet d'obtenir une durée de transit qui correspond à la durée de transit d'une onde de pouls dans l'aorte.

Le calcul du temps de transit de l'onde de pouls dans l'aorte permet ainsi de déterminer la vitesse de propagation de l'onde de pouls dans l'aorte qui est un paramètre physique représentatif de manière fiable et répétable de la rigidité artérielle du sujet.

En effet, l'aorte comprend principalement des tissus élastiques. Ainsi, une éventuelle augmentation de la rigidité artérielle du sujet se traduit au niveau de l'aorte du sujet par une diminution des propriétés élastiques des tissus de l'aorte et donc une augmentation de la vitesse de l'onde de pouls au niveau de l'aorte.

L'impact d'une éventuelle augmentation de rigidité artérielle est ainsi plus important sur l'aorte que sur le circuit de circulation du coeur au doigt et du début de l'artère fémorale à l'orteil, de sorte que la détermination de la vitesse d'onde de pouls au niveau de l'aorte permet de disposer d'un paramètre représentatif de manière fiable et répétable de la rigidité artérielle.

En outre, le fait de positionner le ou chaque capteur à l'extrémité d'un membre pour détecter l'arrivée de l'onde de pouls permet de déterminer de manière fiable et reproductible cet instant d'arrivée de l'onde.

Pour résumer, l'appareil selon l'invention permet de calculer une vitesse, correspondant à celle de l'onde de pouls aortique, en fonction de la différence de temps de transit d'une onde de pouls entre un orteil et un doigt et de la longueur aortique, ce qui permet ensuite d'identifier de manière fiable et répétable une éventuelle augmentation de la rigidité artérielle et par la suite d'évaluer un niveau de risque potentiel lié à cette rigidité, tel qu'un risque cardio-vasculaire.

Selon une caractéristique avantageuse, le calcul de la longueur aortique comprend la multiplication de la taille du sujet par un coefficient dépendant de l'âge du sujet compris dans la plage [0,3;0,4].

Dans un mode de réalisation particulièrement avantageux de l'invention, ledit coefficient varie en fonction de l'âge de telle sorte que ladite longueur aortique augmente de 0,9 à 1,1 cm par décennie supplémentaire, de préférence de 1,0 cm par décennie supplémentaire.

Dans un mode de réalisation particulier de l'invention, ledit coefficient est égal à $(0,336+0,0006*AGE)$ avec l'âge AGE en années.

De façon préférentielle, ladite vitesse d'onde de pouls aortique (Vop_aort) s'exprime sous la forme d'une fonction affine du rapport de ladite longueur aortique (Laort) sur ladite durée de transit aortique (Top_aort).

Le Demandeur a en effet constaté, de façon inédite, que la vitesse d'onde de pouls aortique n'est pas directement proportionnelle au rapport entre la longueur aortique et la durée de transit aortique mais varie de façon affine en fonction du rapport précédent.

Selon une caractéristique avantageuse de l'invention, l'unité de traitement et de calcul comprend des instructions pour calculer la vitesse d'onde de pouls aortique de la manière suivante :

$$Vop\_aort = a_1 * Laort/Top\_aort - b_1 ;$$

avec Vop_aort la vitesse d'onde de pouls aortique en mètre par seconde, Top_aort la durée de transit aortique en seconde, Laort la longueur aortique en mètre, $a_1$ une constante comprise entre 1,25 et 1,65, de préférence sensiblement égale à 1,4 et $b_1$ une constante comprise entre 1, et 1,6, de préférence sensiblement égale à 1,5.

Selon une caractéristique avantageuse de l'invention, ledit appareil comprenant des moyens de mesure de l'activité cardiaque du sujet, tels qu'un électrocardiographe permettant de détecter l'instant de départ de l'onde de pouls, ladite durée de transit aortique est égale à la différence entre la durée séparant l'instant d'arrivée de l'onde de pouls à l'orteil de l'instant de départ de l'onde de pouls, et la durée séparant l'instant d'arrivée de l'onde de pouls au doigt de l'instant de départ de l'onde de pouls.

Selon une caractéristique avantageuse de l'invention, les moyens de mesure de l'activité cardiaque du sujet sont formés par un électrocardiographe apte à générer un électrocardiogramme présentant notamment une variation d'amplitude périodique du signal électrique d'activité cardiaque, appelée complexe QRS, l'unité de traitement et de calcul étant configurée pour déterminer l'instant de départ de l'onde de pouls depuis le coeur à partir de l'onde R dudit complexe QRS, l'unité de traitement et de calcul étant de préférence configurée pour associer l'instant de départ de l'onde de pouls depuis le coeur à l'instant correspondant à la fin de l'onde R du complexe QRS.

Selon un aspect particulier, les deux instants d'arrivée d'onde de pouls sont inclus dans une plage de valeurs de temps d'étendue comprise entre 1 et 200 millisecondes.

L'utilisation d'une plage de temps donnée pour identifier les instants d'arrivée d'onde de pouls, permet de s'assurer que lesdits instants d'arrivée utilisés pour calculer la durée de transit aortique sont bien les instant d'arrivée aux différents membres d'une même onde de pouls, et ainsi permettre de réaliser ledit calcul de durée de transit aortique sans avoir à connaitre l'instant de départ de l'onde de pouls.

Avantageusement, ledit ou chaque capteur d'onde de pouls est formé d'un émetteur et d'un récepteur de lumière, de préférence opérant dans l'infrarouge.

Préférentiellement, le capteur d'onde de pouls est porté par une pince permettant la fixation dudit capteur à l'extrémité du membre. Le fait d'utiliser une pince pour le positionnement du capteur à l'extrémité d'un membre du sujet permet de stabiliser le signal de l'onde de pouls détectée au niveau de ce membre.

L'appareil selon l'invention peut être conçu sous la forme d'un appareil portable de faible encombrement aisé à manipuler, permettant une mesure de la vitesse de l'onde de pouls non invasive, et requérant un minimum d'intervention de la part de l'opérateur.

De préférence l'extrémité du membre choisi est l'extrémité du majeur (doigt le plus long) pour la main, et l'extrémité du deuxième orteil pour le pied (orteil fin le plus long) offrant une meilleure chance de capter la lumière qui traverse la pulpe dudit doigt ou orteil.

L'invention concerne également un procédé de détermination de la vitesse de propagation d'une onde de pression sanguine, dite onde de pouls, d'un sujet, ce procédé comportant les étapes suivantes :

a)- détermination de l'instant d'arrivée d'une onde de pouls à un doigt du sujet au niveau duquel est positionné un capteur dit d'onde de pouls,

b)- détermination de l'instant d'arrivée d'une onde de pouls à un orteil du sujet au niveau duquel est positionné un capteur dit d'onde de pouls,

c)- calcul par une unité de traitement et de calcul d'une valeur de temps, appelée durée de transit aortique, correspondant à la différence entre lesdits instants d'arrivée ;

d)- calcul par ladite unité de traitement et de calcul de ladite vitesse de propagation d'onde de pouls, appelée vitesse d'onde de pouls aortique, en fonction de ladite durée de transit aortique, ledit calcul comprenant la division de la longueur aortique, calculée à partir de la taille et de l'âge du sujet, par ladite durée de transit aortique.

Selon un mode de réalisation particulier, les étapes a) et b) sont réalisées successivement, dans l'ordre ou sens inverse, avec un seul et même capteur d'onde de pouls. Dans ce cas on positionne le capteur d'abord au doigt ou à l'orteil, puis à l'orteil ou au doigt. Dans ce cas l'instant de départ de chaque onde de pouls est détecté à l'aide de moyens de mesure de l'activité cardiaque du sujet.

Selon un autre mode de réalisation particulier, les étapes a) et b) sont réalisées avec deux capteurs d'onde de pouls distincts, l'un positionné au doigt et l'autre à l'orteil.

Selon une caractéristique avantageuse du procédé selon l'invention, la vitesse d'onde de pouls aortique est calculée à l'aide de la formule :

$$Vop\_aort = a_1 * Laort/Top\_aort - b_1,$$

comme expliqué ci-dessus.

Selon une caractéristique avantageuse du procédé selon l'invention, ledit procédé comprend la détermination de l'instant de départ de l'onde de pouls à l'aide de moyens de mesure de l'activité cardiaque du sujet, et ladite durée de transit aortique est égale à la différence entre la durée séparant l'instant d'arrivée de l'onde de pouls à l'orteil de l'instant de départ de l'onde de pouls, et la durée séparant l'instant d'arrivée de l'onde de pouls au doigt de l'instant de départ de l'onde de pouls.

Selon une caractéristique avantageuse du procédé selon l'invention, les deux instants d'arrivée sont inclus dans une plage de valeurs de temps d'étendue comprise entre 1 et 200 millisecondes.

L'invention sera bien comprise à la lecture de la description suivante d'exemples de réalisation, en référence aux dessins annexés dans lesquels :

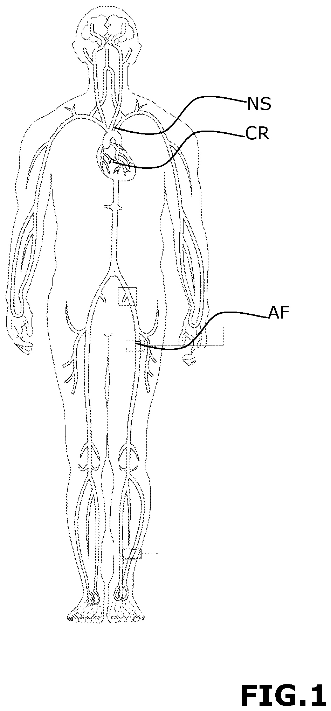

- la figure 1 est une vue schématique du corps d'un sujet montrant les emplacements du noeud sternal, noté NS, et du début de l'artère fémorale, notée AF ;
- la figure 2 est une vue schématique d'un appareil selon un mode de réalisation de l'invention à l'état positionné des capteurs d'onde de pouls à un doigt et à un orteil du sujet, ledit appareil étant dépourvu de moyens de détection d'activité cardiaque ;
- la figure 3 est une vue schématique d'un appareil selon un mode de réalisation de l'invention à l'état positionné de capteurs à un doigt et à un orteil du sujet, et à l'état positionné de moyens de détection d'activité cardiaque sur le corps du sujet ;
- la figure 4 est une vue schématique d'un appareil selon un autre mode de réalisation de l'invention pour lequel ledit appareil comprend des moyens de détection d'activité cardiaque et un seul capteur d'onde de pouls;

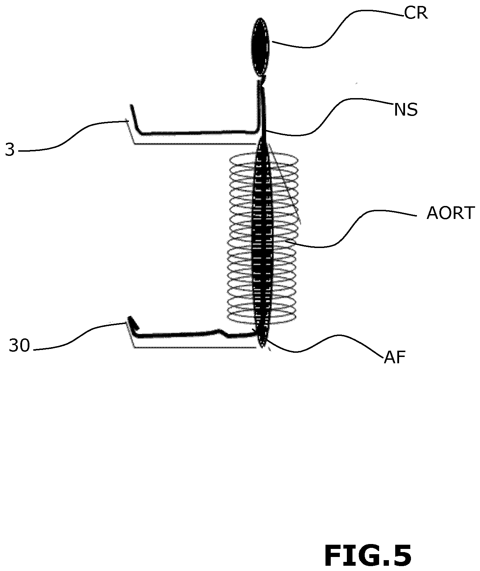

- la figure 5 est une vue schématique du circuit de circulation du sang depuis le coeur, noté CR, jusqu'à un doigt et depuis le coeur jusqu'à un orteil.
- la figure 6 est un schéma de blocs illustrant des étapes du procédé selon un mode de réalisation de l'invention.

En référence aux figures et comme rappelé ci-dessus, l'invention concerne un appareil 1 de détermination de la vitesse de propagation d'une onde de pression sanguine d'un sujet, dite onde de pouls. L'onde de pouls correspond au flux sanguin envoyé par le coeur dans les différentes parties du corps à chaque battement du coeur.

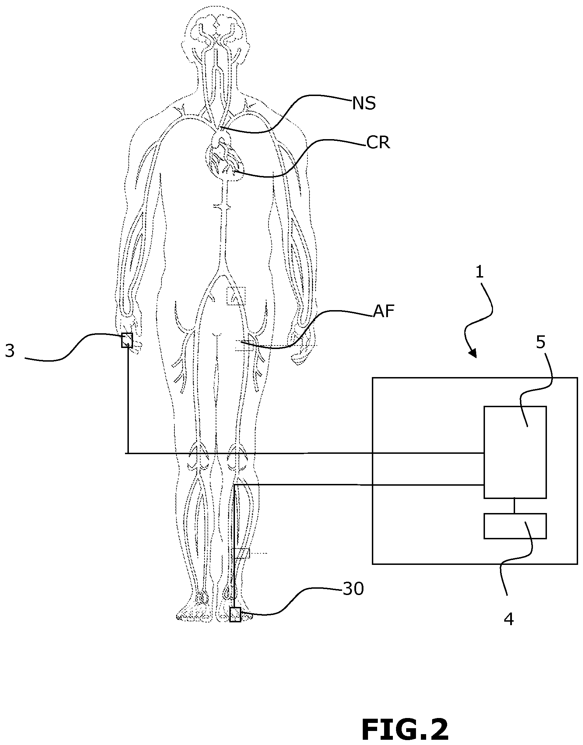

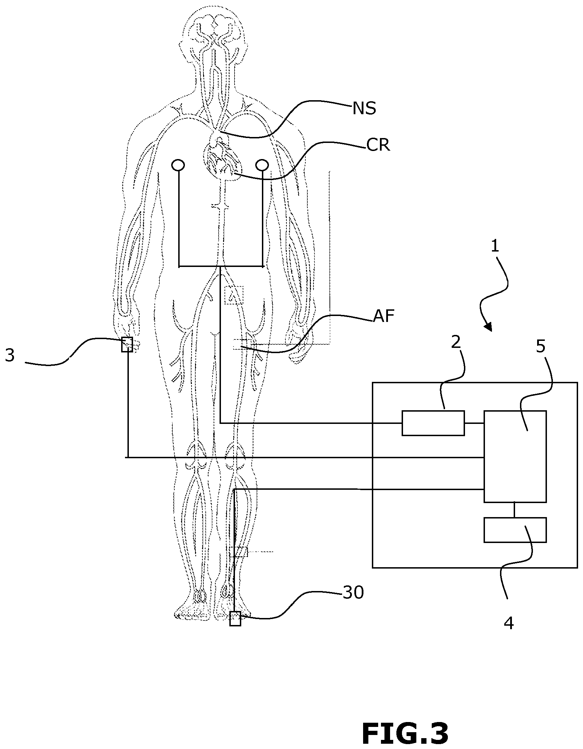

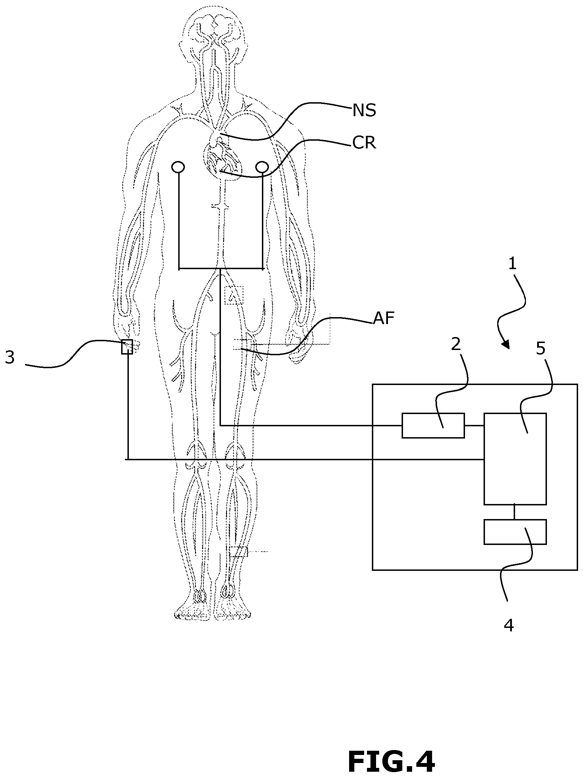

Selon un mode de réalisation de l'invention illustré aux figures 2 et 3, ledit appareil comprend un premier et un deuxième capteur 3, 30 d'onde de pouls. L'un 3 des capteurs est destiné à être positionné à un doigt d'une main et l'autre capteur 30 à un orteil d'un pied du sujet. Comme détaillé ci-après, le mode de réalisation de la figure 3 se distingue de celui de la figure 2 par le fait que dans le mode de réalisation de la figure 3, l'appareil est muni de moyens de mesure de l'activité cardiaque 2 du sujet, tels qu'un électrocardiographe. Selon une variante de réalisation détaillée ci-après et illustrée à la figure 4, on peut prévoir que l'appareil comprend un seul capteur d'onde de pouls.

Le ou chaque capteur 3, 30 d'onde de pouls permet de détecter le passage de l'onde de pouls correspondant à l'onde du flux sanguin envoyée par le coeur dans les différentes parties du corps du sujet et notamment à l'extrémité du membre auquel est positionné ledit capteur.

Le ou chaque capteur 3, 30 d'onde de pouls permet d'acquérir dans le temps l'amplitude d'un paramètre du flux sanguin et/ou du vaisseau sanguin dans lequel circule ledit flux sanguin à l'endroit où est positionné le capteur, ledit paramètre étant représentatif de l'onde de pression formée par ledit flux sanguin.

Le paramètre mesuré peut être un paramètre de pression mesurée via la pression que subit la paroi du vaisseau du fait du passage du flux sanguin à travers ledit vaisseau. Dans ce cas, le capteur d'onde de pouls est un capteur de pression configuré pour mesurer les variations de pression subies par la paroi du vaisseau sanguin en surface duquel le capteur est disposé.

Le paramètre mesuré peut également être un paramètre de réflexion et/ou de transmission d'une lumière par ou à travers le vaisseau sanguin. Dans ce cas, le ou chaque capteur 3 d'onde de pouls est formé d'un émetteur et d'un récepteur de lumière, de préférence infrarouge.

Autrement dit, le ou chaque capteur d'onde de pouls mesure un paramètre dont les variations dans le temps permettent de déterminer l'instant de passage de l'onde de pouls à l'endroit où est positionné ledit capteur d'onde de pouls.

Avantageusement, ledit ou chaque capteur d'onde de pouls est porté par une pince pour permettre un positionnement stable par pincement dudit capteur à l'extrémité du membre choisi du sujet.

En variante, ledit ou chaque capteur d'onde de pouls peut être un capteur à effet Doppler, piézo-électrique ou autre.

Ledit appareil comprend aussi une unité 5 de traitement et de calcul. L'unité comprend une mémoire dans laquelle peuvent être mémorisées des données, notamment la taille, notée H, du sujet et l'âge du sujet. Ladite unité de traitement et de calcul 5 est de préférence formée par un microprocesseur. Lorsque dans la suite de la description, il est précisé que l'unité de traitement et de calcul 5 est configurée pour réaliser une opération donnée, cela signifie que le microprocesseur comprend des instructions informatiques permettant de réaliser ladite opération.

Ladite unité de traitement et de calcul 5 est apte à communiquer avec le ou chaque capteur 3, 30 et les moyens de mesure de l'activité cardiaque 2 lorsqu'ils sont présents. On peut prévoir que le ou les capteur(s) et/ou les moyens de mesure de l'activité cardiaque, communique(nt) de manière filaire ou par ondes radio (Hertziennes) avec l'unité de traitement et de calcul.

L'appareil comprend aussi une interface 4 d'entrée de données configurée pour permettre l'entrée d'au moins la taille H et l'âge du patient. Dans l'exemple illustré aux figures, ladite interface 4 d'entrée de données est une interface homme machine 4 qui comprend par exemple un écran d'affichage et des moyen d'entrée de données, tels que des boutons, clavier ou fenêtre de saisie d'un écran tactile.

En référence à la figure 2 ou 3, ladite unité 5 comprend des instructions pour déterminer, à l'aide du capteur 3 positionné au doigt, l'instant d'arrivée TB d'une onde de pouls audit doigt, et déterminer, à l'aide du capteur 30 positionné à l'orteil, l'instant d'arrivée TC de cette onde de pouls à l'orteil.

Ladite unité 5 calcule ensuite une valeur de temps, appelée durée de transit aortique, notée Top_aort, correspondant à la différence entre lesdits instants d'arrivée à l'orteil et au doigt : TC - TB. On peut prévoir de prendre la valeur absolue de cette différence.

Puis l'unité 5 calcule une vitesse notée Vop_aort, appelée vitesse d'onde de pouls aortique, dépendant du rapport entre la longueur aortique Laort, fonction de la taille, notée H, et de l'âge du sujet, et ladite durée de transit aortique Top_aort.

Avantageusement, le calcul de la longueur aortique Laort comprend la multiplication de la taille (H) du sujet par un coefficient dépendant de l'âge du sujet compris dans la plage de valeurs [0,3;0,4].

Plus précisément, l'unité de traitement et de calcul 5 comprend des instructions pour calculer la vitesse d'onde de pouls aortique de la manière suivante :

$$Vop\_aort = a_1 * Laort/Top\_aort - b_1$$

Avec Vop_aort la vitesse d'onde de pouls aortique en mètre par seconde, Top_aort la durée de transit aortique en seconde, Laort la longueur aortique en mètre, $a_1$ une constante égale à 1,43 et $b_1$ une constante égale à 1,53.

Dans ce mode de réalisation particulier de l'invention, la longueur aortique est calculée à l'aide de la formule suivante : Laort (m) =(0,336+0,0006*AGE)*H, où l'âge AGE est exprimé en années et la taille H en mètres.

Dans une variante, la vitesse d'onde de pouls peut être calculée par la formule suivante :

$$Vop\_aort = ((H*K1/ Top\_aort)-a)/b + c$$

Avec Vop_aort : la vitesse d'onde de pouls aortique en mètre par seconde (m/s)
Top_aort : durée de transit aortique en seconde (s)
H : la taille du sujet en mètre (m)
K1 un coefficient dépendant de l'âge du sujet compris entre 0,3 et 0,4, et a, b et c trois constantes comprises respectivement entre 2 et 4 m/s, entre 0,6 et 0,8, et entre 2 et 3 m/s, de préférence sensiblement égales à 3, 0,7 et 2,76 respectivement.

La longueur du circuit de circulation de sang parcourue par l'onde de pouls depuis le coeur CR, au niveau des valves ou sigmoïdes aortiques, jusqu'à l'orteil, est formée principalement de la distance du coeur CR au noeud sternal NS, de la longueur du noeud sternal NS au début de l'artère

fémorale AF (correspondant à la longueur de l'aorte AORT), et de la longueur du début de l'artère fémorale AF à l'orteil.

En outre, la longueur du circuit de circulation de sang parcourue par l'onde de pouls depuis le coeur CR jusqu'au doigt est formée principalement de la distance du coeur CR au noeud sternal NS et de la longueur du noeud sternal NS au doigt.

Or, on observe que la longueur du début de l'artère fémorale AF à l'orteil correspond sensiblement à la longueur du noeud sternal NS au doigt.

En outre, le circuit de circulation de sang du coeur CR au doigt et le circuit de circulation de sang du début de l'artère fémorale AF à l'orteil sont principalement formés de tissus musculaires. Ainsi, la vitesse de propagation d'onde de pouls est sensiblement la même le long de ces deux circuits.

Il en résulte que le fait de calculer une valeur de temps correspondant à la différence entre l'instant d'arrivée TC de l'onde de pouls à l'orteil et l'instant d'arrivée TB de l'onde de pouls au doigt permet d'obtenir une durée de transit qui correspond à la durée de transit de l'onde de pouls dans l'aorte AORT.

Le calcul du temps de transit de l'onde de pouls dans l'aorte permet ainsi de déterminer une vitesse d'onde de pouls dans l'aorte qui, comme expliqué ci-dessus, est un paramètre physique représentatif de manière fiable et répétable de la rigidité artérielle du sujet.

Comme rappelé ci-dessus, selon le mode de réalisation particulier de l'invention illustré à la figure 2, l'appareil ne comprend pas de moyens de mesure de l'activité cardiaque du sujet permettant de détecter l'instant de départ de l'onde de pouls, de sorte que l'unité 5 vérifie que les deux instants d'arrivée TB, TC d'onde de pouls sont inclus dans une fenêtre ou plage de valeurs de temps d'étendue comprise entre 1 et 200 millisecondes, afin de s'assurer que les deux instants d'arrivée TB, TC correspondent bien à une même onde de pouls.

Selon le mode de réalisation particulier de l'invention illustré à la figure 3, ledit appareil comprend des moyens de mesure de l'activité cardiaque 2 du sujet, tels qu'un électrocardiographe. Les moyens de mesure de l'activité cardiaque 2 du sujet sont formés par un électrocardiographe apte à générer un électrocardiogramme présentant notamment une variation d'amplitude périodique du signal électrique d'activité cardiaque, appelée complexe QRS. L'unité de traitement et de calcul 5 est configurée pour déterminer l'instant de départ de l'onde de pouls depuis le coeur à partir de l'onde R dudit complexe QRS. Pour plus de détails, l'Homme du Métier consultera la demande de brevet publiée sous le numéro FR2947167 qui décrit des exemples de méthode de détermination de l'instant de départ de l'onde de pouls depuis le coeur à partir de l'onde R dudit complexe QRS.

Ainsi, selon le mode de réalisation illustré à la figure 3, ladite unité 5 est configurée pour détecter l'instant de départ TA de l'onde de pouls à l'aide desdits moyens de mesure de l'activité cardiaque 2 du sujet. Ladite durée de transit aortique Top_aort est égale à la différence entre la durée, notée DELTAC, séparant l'instant d'arrivée de l'onde de pouls à l'orteil TC de l'instant de départ TA de l'onde de pouls, et la durée, notée DELTATB, séparant l'instant d'arrivée de l'onde de pouls TB au doigt de l'instant de départ TA de l'onde de pouls.

Dans l'exemple illustré aux figures 2 et 3, l'appareil comprend deux capteurs 3, 30 d'onde de pouls, ce qui permet de déterminer les instants d'arrivés au doigt et à l'orteil d'une même onde de pouls, et donc la durée de transit aortique avec ou sans la connaissance de l'instant de départ de ladite onde de pouls. Ainsi, les ondes de pouls mesurées dans un membre supérieur (main) et dans un membre inférieur (pied) proviennent d'une même onde sanguine émise par le coeur, ce qui améliore la fiabilité du calcul.

En variante, comme illustré à la figure 4, on peut prévoir que l'appareil soit équipé d'un seul capteur d'onde de pouls 3 qui peut être destiné à être positionné au doigt pour mesurer l'instant d'arrivée d'une onde de pouls au doigt puis à l'orteil pour mesurer l'instant d'arrivée d'une autre onde de pouls à l'orteil, ou inversement.

L'appareil permet de mettre en oeuvre un procédé de détermination de la vitesse de propagation de l'onde de pouls aortique d'un sujet de la manière suivante. Le ou chaque capteur d'onde de pouls est maintenu par une pince à l'extrémité d'un orteil ou d'un doigt. La taille H et l'âge du sujet sont rentrés dans l'appareil via l'interface 4 d'entrée correspondante.

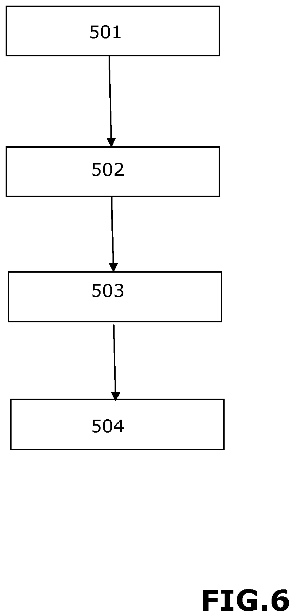

Comme illustré à la figure 6, à l'étape 501, l'unité 5 détermine l'instant d'arrivée TB d'une onde de pouls à un doigt d'une main du sujet au niveau duquel est positionné un capteur d'onde de pouls, et, à l'étape 502, l'instant d'arrivée TC d'une onde de pouls à un orteil du sujet au niveau duquel est positionné un capteur d'onde de pouls. L'instant d'arrivée d'une onde de pouls peut être déterminé à partir de différents points remarquables de l'onde tels que : pied de l'onde, point d'inflexion de la phase ascendante de l'onde, le sommet de l'onde, ou le point d'intersection de la tangente de la courbe ascendante de l'onde avec la ligne de base, ou encore le centre de gravité de l'onde (c'est-à dire le centre de gravité de la surface définie par la forme de la courbe de l'onde).

Comme expliqué ci-dessus, le capteur positionné au doigt et le capteur positionné à l'orteil peuvent être formé par un unique capteur déplacé du doigt à l'orteil ou inversement. Lorsque l'appareil utilise deux capteurs distincts, l'instant d'arrivée TB et l'instant d'arrivée TC peuvent être les instants d'arrivée d'une même onde de pouls, alors que dans le cas d'un capteur positionné successivement à l'extrémité d'un membre puis d'un autre, les mesures sont décalées dans le temps de sorte que l'onde de pouls associée à l'instant d'arrivée TB déterminé est une onde de pouls générée à un instant différent de celle associée à l'instant d'arrivée TC déterminé.

Dans le cas où les instants TB et TC sont associés à une même onde de pouls, il est possible de calculer (étape 503) la durée de transit aortique Top_aort, en faisant simplement la différence entre lesdits instants d'arrivée TB, TC, puisque l'instant de départ est le même. Préférentiellement, l'unité 5 vérifie que les deux instants d'arrivée TB, TC sont inclus dans une fenêtre ou plage de valeurs de temps d'étendue comprise entre 1 et 200 millisecondes.

A l'inverse lorsque les instants TB et TC sont associées à deux ondes de pouls générées à des instant différents, il convient de calculer (étape 503) la durée de transit aortique Top_aort, en tenant compte des instants de départ de chaque onde de pouls comme expliqué ci-dessus.

Puis, à l'étape 504, la vitesse Vop_aort, appelée vitesse d'onde de pouls aortique, est calculée à partir de la division d'une valeur, appelée longueur aortique Laort, à partir de la taille (H) et de l'âge du sujet, par ladite durée de transit aortique Top_aort.

Préférentiellement, on applique la formule :

$$Vop\_aort = a_1*Laort/Top\_aort - b_1,$$

comme détaillé ci-dessus.

Dans le cas où l'on utilise un appareil muni d'un électrocardiographe pour déterminer l'instant de départ de chaque onde de pouls au doigt et à l'orteil, ledit électrocardiographe peut être utilisée de la manière suivante. On applique les deux électrodes de l'électrocardiographe sur deux zones distinctes du corps du sujet. Pour ce faire, il est possible de prévoir que le sujet pose ses deux pouces sur les deux électrodes correspondantes de l'électrocardiographe de l'appareil.

Lors de l'acquisition par l'unité 5 de l'appareil des signaux d'onde de pouls, et éventuellement des signaux d'activité cardiaque, on peut prévoir que ces signaux soient pré-conditionnés, puis soumis à un filtrage analogique et à une amplification. L'unité 5 peut alors procéder à une opération de numérisation et de filtrage numérique pour obtenir en sortie des données transmises à un programme de calcul interne à l'appareil ou exécuté sur un dispositif externe, tel qu'un ordinateur de bureau, portable, tablette, ou smartphone. Une fois la taille et l'âge du sujet rentrés dans ce programme, celui-ci peut calculer, éventuellement en liaison par Internet avec un centre d'analyse et de calcul de données, la vitesse d'onde de pouls aortique comme expliqué ci-dessus. Le résultat du calcul peut être affiché sur l'appareil ou sur un dispositif externe.

**Revendications**

**1.** Appareil (1) de détermination de la vitesse de propagation d'une onde de pression sanguine d'un sujet, dite onde de pouls, ledit appareil comprenant, soit un capteur (3) d'onde de pouls, soit un premier et un deuxième capteur (3,30) d'onde de pouls, ledit ou chaque capteur (3,30) étant positionnable à un doigt d'une main ou à un orteil d'un pied du sujet, ledit appareil comprenant aussi une unité (5) de traitement et de calcul configurée pour :

- déterminer l'instant d'arrivée (TB) d'une onde de pouls à un doigt du sujet, lorsque ledit (3) ou l'un desdits capteur(s) (3, 30) est positionné audit doigt ;
- déterminer l'instant d'arrivée (TC) d'une onde de pouls à un orteil du sujet, lorsque ledit (3) ou l'un desdits capteur(s) (30, 3) est positionné audit orteil ;
- calculer une valeur de temps, appelée durée de transit aortique (Top_aort), correspondant à la différence entre lesdits instants d'arrivée (TC, TB);
- calculer ladite vitesse de propagation d'onde de pouls, appelée vitesse d'onde de pouls aortique (Vop_aort), en fonction de ladite durée de transit aortique (Top_aort), ledit calcul comprenant la division de la longueur aortique (Laort), calculée par ladite unité de traitement et de calcul à partir de la taille (H) et de l'âge du sujet, par ladite durée de transit aortique (Top_aort),

ledit appareil comprenant en outre lorsqu'il comprend un seul capteur d'onde de pouls, des moyens de mesure de l'activité cardiaque (2) du sujet destinés à permettre une détection de l'instant de départ (TA) de l'onde de pouls.

**2.** Appareil (1) selon la revendication 1, **caractérisé en ce que** ladite longueur aortique (Laort) est obtenue en multipliant la taille (H) du sujet par un coefficient fonction de l'âge du sujet, compris dans la plage [0,3;0,4].

**3.** Appareil selon la revendication 2, **caractérisé en ce que** ledit coefficient varie en fonction de l'âge de telle sorte que ladite longueur aortique augmente de 0,9 à 1,1 cm par décennie supplémentaire, de préférence de 1,0 cm par décennie supplémentaire.

**4.** Appareil selon l'une quelconque des revendications 1 à 3, **caractérisé en ce que** ladite vitesse d'onde de pouls aortique (Vop_aort) s'exprime sous la forme d'une fonction affine du rapport de ladite longueur aortique (Laort) sur ladite durée de transit aortique (Top_aort).

**5.** Appareil (1) selon la revendication 4, **caractérisé en ce que** ladite vitesse d'onde de pouls aortique s'exprime de la manière suivante :

$$Vop\_aort = a_1 * Laort / Top\_aort - b_1 \; ;$$

avec Vop_aort la vitesse d'onde de pouls aortique en mètre par seconde, Top_aort la durée de transit aortique en seconde, Laort la longueur aortique en mètre, $a_1$ une constante sensiblement égale à 1,4, et $b_1$ une constante sensiblement égale à 1,5.

6. Appareil (1) selon l'une des revendications précédentes, **caractérisé en ce que** ledit appareil comprenant un seul capteur d'onde de pouls, ladite durée de transit aortique (Top_aort) est égale à la différence entre la durée (DELTATC) séparant l'instant d'arrivée de l'onde de pouls à l'orteil (TC) de l'instant de départ (TA) de l'onde de pouls, et la durée (DELTATB) séparant l'instant d'arrivée de l'onde de pouls au doigt (TB) de l'instant de départ (TA) de l'onde de pouls.

7. Appareil (1) selon l'une des revendications précédentes, **caractérisé en ce que** ledit ou chaque capteur (3, 30) d'onde de pouls est formé d'un émetteur et d'un récepteur de lumière, de préférence opérant dans l'infrarouge.

8. Procédé de détermination de la vitesse de propagation d'une onde de pression sanguine, dite onde de pouls, d'un sujet, le procédé comportant les étapes suivantes :

   a)- détermination de l'instant d'arrivée (TB) d'une onde de pouls à un doigt du sujet au niveau duquel est positionné un capteur dit d'onde de pouls (3 ; 3,30),
   b)- détermination de l'instant d'arrivée (TC) d'une onde de pouls à un orteil du sujet au niveau duquel est positionné un capteur dit d'onde de pouls (3 ; 30, 3),
   c)- calcul par une unité de traitement et de calcul d'une valeur de temps, appelée durée de transit aortique (Top_aort), correspondant à la différence entre lesdits instants d'arrivée (TB, TC);
   d)- calcul par ladite unité de traitement et de calcul de ladite vitesse de propagation d'onde de pouls, appelée vitesse d'onde de pouls aortique (Vop_aort), en fonction de ladite durée de transit aortique (Top_aort) comprenant la division de la longueur aortique (Laort),

   calculée à partir de la taille (H) et de l'âge du sujet, par ladite durée de transit aortique (Top_aort).

9. Procédé selon la revendication 8, **caractérisé en ce que** les étapes a) et b) sont réalisées successivement, dans l'ordre ou sens inverse, avec un seul et même capteur d'onde de pouls (3).

10. Procédé selon la revendication 8, **caractérisé en ce que** les étapes a) et b) sont réalisées avec deux capteurs d'onde de pouls distincts (3,30), l'un (3) positionné au doigt et l'autre à l'orteil (30).

11. Procédé selon l'une des revendications 8 à 10, **caractérisé en ce que** la vitesse d'onde de pouls aortique est calculée à l'aide de la formule :

$$Vop\_aort = a_1 . Laort / Top\_aort - b_1 \; ;$$

avec Vop_aort la vitesse d'onde de pouls aortique en mètre par seconde, Top_aort la durée de transit aortique en seconde, Laort la longueur aortique en mètre, $a_1$ une constante sensiblement égale à 1,4 et $b_1$ une constante sensiblement égale à 1,5.

12. Procédé selon l'une des revendications 8 à 11, **caractérisé en ce que** ledit procédé comprend la détermination de l'instant de départ (TA) de l'onde de pouls à l'aide de moyens de mesure de l'activité cardiaque (2) du sujet, et **en ce que** ladite durée de transit aortique (Top_aort) est égale à la différence entre la durée (DELTATC) séparant l'instant d'arrivée de l'onde de pouls (TC) à l'orteil de l'instant de départ (TA) de l'onde de pouls, et la durée (DELTATB) séparant l'instant d'arrivée de l'onde de pouls (TB) au doigt de l'instant de départ (TA) de l'onde de pouls.

13. Procédé selon l'une des revendications 8 à 12, **caractérisé en ce que** les deux instants d'arrivée (TB, TC) sont inclus dans une plage de valeurs de temps d'étendue comprise entre 1 et 200 millisecondes.

**Patentansprüche**

1. Vorrichtung (1) zur Bestimmung der Ausbreitungsgeschwindigkeit einer Blutdruckwelle eines Menschen, genannt Pulswelle, wobei die Vorrichtung entweder einen Pulswellensensor (3) oder einen ersten und einen zweiten Pulswellensensor (3, 30) umfasst, wobei der oder jeder Sensor (3, 30) an einem Finger einer Hand oder an einem Zeh eines Fußes des Menschen positionierbar ist, wobei die Vorrichtung auch eine Verarbeitungs- und Recheneinheit (5) umfasst, die konfiguriert ist, um:

   - den Moment des Eintreffens (TB) einer Pulswelle in einem Finger des Menschen zu bestimmen, wenn der (3) oder einer der Sensor(en) (3, 30) am Finger positioniert ist;
   - den Moment des Eintreffens (TC) einer Pulswelle in einem Zeh des Menschen zu bestimmen, wenn der (3) oder einer der Sensor(en) (30, 3) am Zeh positioniert ist;
- einen Zeitwert, bezeichnet als Dauer des Aortatransits (Top_aort), zu berechnen, welcher der Differenz zwischen den Momenten des Eintreffens (TC, TB) entspricht;
- die Pulswellen-Ausbreitungsgeschwindigkeit, bezeichnet als Aortapulswellengeschwindigkeit (Vop_aort), in Abhängigkeit von der Dauer des Aortatransits (Top_aort) zu berechnen, wobei die Berechnung die Division der Aortalänge (Laort), berechnet von der Verarbeitungs- und Recheneinheit ausgehend von der Größe (H) und des Alters des Menschen, durch die Dauer des Aortatransits (Top_aort) umfasst,

wobei die Vorrichtung ferner, wenn sie einen einzigen Pulswellensensor umfasst, Messmittel der Herzaktivität (2) des Menschen umfasst, die bestimmt sind, eine Ermittlung des Moments des Startens (TA) der Pulswelle zu erlauben.

2. Vorrichtung (1) nach Anspruch 1, **dadurch gekennzeichnet, dass** die Aortalänge (Laort) durch Multiplizieren der Größe (H) des Menschen mit einem vom Alter des Menschen abhängigen Koeffizienten erhalten wird, der im Bereich [0,3; 0,4] liegt.

3. Vorrichtung nach Anspruch 2, **dadurch gekennzeichnet, dass** der Koeffizient in Abhängigkeit vom Alter derart schwankt, dass die Aortalänge von 0,9 bis 1,1 cm je zusätzliches Jahrzehnt, vorzugsweise um 1,0 cm je zusätzliches Jahrzehnt, zunimmt.

4. Vorrichtung nach einem der Ansprüche 1 bis 3, **dadurch gekennzeichnet, dass** die Aortapulswellengeschwindigkeit (Vop_aort) in Form einer affinen Funktion des Verhältnisses der Aortalänge (Laort) zur Dauer des Aortatransits (Top_aort) ausgedrückt wird.

5. Vorrichtung (1) nach Anspruch 4, **dadurch gekennzeichnet, dass** die Aortapulswellengeschwindigkeit folgendermaßen ausgedrückt wird:

$$Vop\_aort = a_1 * Laort/Top\_aort - b_1;$$

mit Vop_aort als Aortapulswellengeschwindigkeit in Meter je Sekunde, Top_aort als Dauer des Aortatransits in Sekunden, Laort als Aortalänge in Meter, $a_1$ einer Konstanten, die etwa gleich 1,4 ist und $b_1$ einer Konstanten, die etwa gleich 1,5 ist.

6. Vorrichtung (1) nach einem der vorangehenden Ansprüche, **dadurch gekennzeichnet, dass** die Vorrichtung einen einzigen Pulswellensensor umfasst, wobei die Dauer des Aortatransits (Top_aort) gleich der Differenz zwischen der Dauer (DELTATC) ist, welche den Moment des Eintreffens der Pulswelle im Zeh (TC) vom Moment des Startens (TA) der Pulswelle trennt, und der Dauer (DELTATB), welche den Moment des Eintreffens der Pulswelle im Finger (TB) vom Moment des Startens (TA) der Pulswelle trennt.

7. Vorrichtung (1) nach einem der vorangehenden Ansprüche, **dadurch gekennzeichnet, dass** der oder jeder Pulswellensensor (3, 30) von einem Lichtsender und -empfänger gebildet ist, der vorzugsweise im Infrarot arbeitet.

8. Verfahren zur Bestimmung der Ausbreitungsgeschwindigkeit einer Blutdruckwelle, genannt Pulswelle, eines Menschen, wobei das Verfahren die folgenden Schritte umfasst:

a) Bestimmen des Moments des Eintreffens (TB) einer Pulswelle in einem Finger des Menschen, an dem ein Pulswellensensor (3; 3, 30) positioniert ist,
b) Bestimmen des Moments des Eintreffens (TC) einer Pulswelle in einem Zeh des Menschen, an dem ein Pulswellensensor (3; 30, 3) positioniert ist,
c) Berechnen, durch eine Verarbeitungs- und Recheneinheit, eines Zeitwerts, bezeichnet als Dauer des Aortatransits (Top_aort), welcher der Differenz zwischen den Momenten des Eintreffens (TB, TC) entspricht;
d) Berechnen, durch die Verarbeitungs- und Recheneinheit, der Pulswellen-Ausbreitungsgeschwindigkeit, bezeichnet als Aortapulswellengeschwindigkeit (Vop_aort), in Abhängigkeit von der Dauer des Aortatransits (Top_aort), umfassend die Division der Aortalänge (Laort), berechnet ausgehend von der Größe (H) und des Alters des Menschen, durch die Dauer des Aortatransits (Top_aort).

9. Verfahren nach Anspruch 8, **dadurch gekennzeichnet, dass** die Schritte a) und b) aufeinanderfolgend in der Reihenfolge oder umgekehrt mit einem einzigen und demselben Pulswellensensor (3) durchgeführt werden.

10. Verfahren nach Anspruch 8, **dadurch gekennzeichnet, dass** die Schritte a) und b) mit zwei unterschiedlichen Pulswellensensoren (3, 30), einem (3), der am Finger positioniert ist und der andere am Zeh (30), durchgeführt werden.

11. Verfahren nach einem der Ansprüche 8 bis 10, **dadurch gekennzeichnet, dass** die Aortapulswellengeschwindigkeit mit Hilfe der Formel berechnet wird:

$$\text{Vop\_aort} = a_1.\text{Laort}/\text{Top\_aort} - b_1;$$

mit Vop_aort als Aortapulswellengeschwindigkeit in Meter je Sekunde, Top_aort als Dauer des Aortatransits in Sekunden, Laort als Aortalänge in Meter, $a_1$ einer Konstanten, die etwa gleich 1,4 ist und $b_1$ einer Konstanten, die etwa gleich 1,5 ist.

12. Verfahren nach einem der Ansprüche 8 bis 11, **dadurch gekennzeichnet, dass** das Verfahren das Bestimmen des Moments des Startens (TA) der Pulswelle mit Hilfe von Messmitteln der Herzaktivität (2) des Menschen umfasst und dass die Dauer des Aortatransits (Top_aort) gleich der Differenz zwischen der Dauer (DELTATC) ist, welche den Moment des Eintreffens der Pulswelle (TC) im Zeh vom Moment des Startens (TA) der Pulswelle trennt, und der Dauer (DELTATB), welche den Moment des Eintreffens der Pulswelle (TB) im Finger vom Moment des Startens (TA) der Pulswelle trennt.

13. Verfahren nach einem der Ansprüche 8 bis 12, **dadurch gekennzeichnet, dass** die zwei Momente des Eintreffens (TB, TC) in einem Zeitumfangs-Wertebereich inbegriffen sind, der zwischen 1 und 200 Millisekunden liegt.

**Claims**

1. An apparatus (1) for determining speed of propagation rate of a blood pressure wave of a subject, known as a pulse wave, said apparatus comprising either a pulse wave sensor (3) or a first and a second pulse wave sensor (3, 30), said or each sensor (3, 30) being able to be positioned at a finger of one hand or a toe of one foot of the subject, said apparatus also including a processing and calculation unit (5) configured for:

   - determining an arrival time (TB) of a pulse wave at a finger of the subject, when said sensor (3) or one of said sensor(s) (3, 30) is positioned at said finger;
   - determining an arrival time (TC) of a pulse wave at a toe of the subject, when said sensor (3) or one of said sensor(s) (30, 3) is positioned at said toe;
   - calculate a time value, called aortic transit time (Top_aort), corresponding to the difference between said arrival times (TC, TB);
   - calculating said pulse wave propagation speed, so-called aortic pulse wave velocity (Vop_aort), as a function of said aortic transit time (Top aort), said calculation comprising dividing the aortic length (Laort), calculated by said processing and calculation unit from the size (H) and age of the subject, by said aortic transit time (Top_aort),

   said apparatus further comprising, when it comprises a single pulse wave sensor, means for measuring the cardiac activity (2) of the subject intended to allow detection of the pulse wave start time (TA).

2. The apparatus (1) according to claim 1, **characterised in that** said aortic length (Laort) is obtained by multiplying the size (H) of the subject by a coefficient depending on the age of the subject, within the range [0.3; 0.4].

3. The apparatus according to claim 2, **characterised in that** said coefficient varies with age such that said aortic length increases from 0.9 to 1.1 cm per additional decade, preferably by 1.0 cm per additional decade.

4. The apparatus according to any of claims 1 to 3, **characterised in that** said aortic pulse wave velocity (Vop_aort) is expressed as an affine function of the ratio of said aortic length (Laort) to said aortic transit time (Top_aort).

5. Apparatus (1) according to claim 4, **characterised in that** said speed of aortic pulse wave is expressed as follows:

$$\text{Vop\_aort} = a_1*\text{Laort}/\text{Top\_aort} - b_1;$$

with Vop_aort the aortic pulse wave velocity in meters per second, Top_aort the aortic transit time in seconds, Laort the aortic length in meters, $a_1$ is a constant substantially equal to 1.4, and $b_1$ a constant substantially equal to 1.5.

6. The apparatus (1) according to one of the preceding claims, **characterised in that** said apparatus comprising a single pulse wave sensor, said aortic transit time (Top_aort) is equal to the difference between the time (DELTATC) separating the arrival time of the pulse wave at the toe (TC) from the departure time (TA) of the pulse wave, and the time (DELTATB) separating the time of arrival of the pulse wave at the finger (TB) from the time of departure (TA) of the pulse wave.

7. The apparatus (1) according to one of the above claims, **characterised in that** said or each pulse wave sensor (3, 30) is formed by a light transmitter and receiver, preferably operating in the infrared range.

8. A method for determining the propagation speed of a blood pressure wave, kwnown as a pulse wave, of a subject, the method comprising the following steps:

   a)- determination of an arrival time (TB) of a pulse wave at a finger of the subject at which a so-called pulse wave sensor (3; 3,30) is positioned,

   b)- determination of an arrival time (TC) of a pulse wave at a toe of the subject at which a so-called pulse wave sensor (3; 30,3) is positioned,

   c)- calculation by a processing and calculation unit of a time value, so-called aortic transit time (Top_aort), corresponding to the difference between said arrival times (TB, TC);

   d)- calculation by said processing and calculation unit of said pulse wave propagation speed, so-called aortic pulse wave velocity (Vop_aort), as a function of said aortic transit time (Top_aort) comprising dividing the aortic length (Laort), calculated from the size (H) and age of the subject, by said aortic transit time (Top_aort).

9. A method according to claim 8, **characterised in that** steps a) and b) are carried out successively, in order or in the opposite direction, with a single pulse wave sensor (3).

10. The method according to claim 8, **characterised in that** steps a) and b) are carried out with two separate pulse wave sensors (3, 30), one (3) positioned on the finger and the other on the toe (30).

11. The method according to one of claims 8 to 10, **characterised in that** the aortic pulse wave velocity is calculated using the formula:

$$Vop\_aort = a_1.Laort/Top\_aort - b_1 \ ;$$

   with Vop_aort the aortic pulse wave velocity in meters per second, Top_aort the aortic transit time in seconds, Laort the aortic length in meters, $a_1$ is a constant substantially equal to 1.4 and $b_1$ a constant substantially equal to 1.5.

12. The method according to one of claims 8 to 11, **characterised in that** said method comprises determining the starting time (TA) of the pulse wave using means for measuring the cardiac activity (2) of the subject, and **in that** said aortic transit time (Top_aort) is equal to the difference between the time (DELTATC) separating the arrival time of the pulse wave (TC) at the toe from the departure time (TA) of the pulse wave, and the time (DELTATB) separating the arrival time of the pulse wave (TB) at the finger from the departure time (TA) of the pulse wave.

13. The method according to one of claims 8 to 12, **characterised in that** the two arrival times (TB, TC) are included in a range of time values with a range of between 1 and 200 milliseconds.

**FIG.1**

**FIG.2**

**FIG.3**

**FIG.4**

**FIG.5**

FIG.6

## RÉFÉRENCES CITÉES DANS LA DESCRIPTION

**Documents brevets cités dans la description**

- FR 2947167
- US 2008027330 A1

**Littérature non-brevet citée dans la description**

- Pulse Wave Velocity and Digital Volume Pulse as indirect estimators of blood pressure: pilot study on healthly volunteers. **PADILLA et al.** cardiovascular engineering. Kluwer academic publishers- Plenum publishers, 2009, vol. 9
- **HALLAB et al.** un nouvel index pour évaluer le vieillissement artériel indépendamment de la pression artérielle : pOpscore. *Annales de cardiologie et d'angéiologie,* 2012, vol. 61 (3
- The difference in pulse transit time to the toe and finger measured by photoplethysmography: difference in pulse transit time to the toe and finger. **NITZAN et al.** Physiological measurement. institute of physics publishing, 2001, vol. 23